# FASCICULE DE BREVET EUROPEEN

(11) **EP 4 641 587 B1**
(45) Date de publication et mention de la délivrance du brevet: **19.08.2026**
(21) Numéro de dépôt: 25171498.6
(22) Date de dépôt: 18.04.2025
(51) Int. Cl.: G21F 3/02, G02C 1/06, G02C 5/00, G02C 9/04, A61F 9/02, G02C 7/16

(54) **MONTURE POUR LUNETTES DE PROTECTION CONTRE LES RAYONNEMENTS IONISANTS**
BRILLENGESTELL ZUM SCHUTZ GEGEN IONISIERENDE STRAHLUNG
FRAME FOR GLASSES PROTECTING AGAINST IONISING RADIATION

(30) Priorité: 24.04.2024 FR 2404235
(43) Date de publication de la demande: 29.10.2025
(73) Titulaire: NOIXX, 07400 Alba-la-Romaine (FR)
(72) Inventeur: COULANGE, Florian, 07400 Alba-la-Romaine (FR)
(74) Mandataire: Loyer & Abello

(56) Documents cités:
- WO-A1-2020/229701
- CN-A- 117 860 476
- JP-B2- 7 120 569

## Description

### Domaine technique

L'invention se rapporte au domaine de la protection individuelle contre les rayonnements ionisants. Plus particulièrement, l'invention concerne le domaine des montures de protection contre les rayonnements ionisants et des mesures de doses radioactives associées.

### Arrière-plan technologique

Il existe une pluralité de dispositifs de protection individuels permettant de protéger des parties du corps d'un agent des rayonnements ionisants présents dans son environnement. Ces dispositifs de protections sont importants en ce qu'ils permettent de réduire les risques d'effets néfastes pour la santé de l'agent pouvant être la conséquence d'une exposition aux rayonnements ionisants. Les effets néfastes sont par exemple : les brûlures cutanées, le syndrome de l'irradiation aigu (SIA) ou le cancer. En effet, au-delà de certains seuils, les rayonnements ionisants peuvent altérer le fonctionnement des tissus et/ou des organes.

Le document FR3080215A1 divulgue par exemple des lunettes de protection contre les rayonnements ionisants comprenant une monture de lunettes de protection contre les rayonnements ionisants, comprenant une partie frontale se prolongeant latéralement par deux éléments de protection latérale. La partie frontale et les éléments de protection latérale sont réalisé en un matériau radio-atténuateur.

Une telle paire de lunettes permet de réduire la dose de rayonnements ionisants reçue par l'utilisateur aux niveaux du visage mais ne permet pas de mesurer la dose de rayonnements ionisants reçue par l'utilisateur sans ajouter des dispositifs auxiliaires de mesures qui sont encombrants et qui ne reflètent pas avec précision la dose effectivement reçue par les yeux de l'utilisateur.

Le document WO 2020/229701 A1 décrit une monture pour lunettes de protection contre les rayonnements ionisants, la monture comprenant un porte dosimètre comportant une chambre de réception destinée à recevoir un dosimètre.

Or, les dispositifs de protections doivent permettent à l'agent de travailler dans de bonnes conditions, c'est-à-dire avec sécurité et confort. Ainsi, il convient de résoudre les problèmes susmentionnés.

### Résumé de l'invention

L'invention fournit une monture pour lunettes de protection contre les rayonnements ionisants, la monture (1, 101) comprenant une partie frontale (2, 102), une première partie latérale (3, 103) et une deuxième partie latérale (4, 104) s'étendant respectivement depuis une première et une deuxième extrémités latérales (25, 26, 125, 126) de la partie frontale, dans laquelle la monture comprend une première charge capable d'absorber des rayonnements ionisants, dans laquelle la partie frontale comporte un premier logement (5, 105) qui est destiné à porter un premier verre minéral et un deuxième logement (6, 106) qui est destiné à porter un deuxième verre minéral, le premier et le deuxième logements étant espacés l'un de l'autre par une zone de pont (7, 107) de la partie frontale,dans laquelle la monture comprend un porte dosimètre (9, 109), le porte dosimètre comportant une chambre de réception (91, 191) destinée à recevoir un dosimètre, le porte dosimètre étant saillant derrière la partie frontale dans le prolongement du premier logement ou du deuxième logement, de sorte que la chambre de réception et le dosimètre soient situés entre le premier logement ou le deuxième logement et les yeux de l'utilisateur,dans laquelle le porte dosimètre est saillant depuis la première partie latérale ou depuis la deuxième partie latérale depuis une zone d'angle formée par l'intersection d'une surface interne (29, 129) de la partie frontale et d'une surface interne (31, 131) de la première partie latérale ou de la deuxième partie latérale, dans laquelle la première partie latérale ou la deuxième partie latérale comporte une zone de fixation (40) pour porte dosimètre, le porte dosimètre étant fixé à la zone de fixation de manière amovible par un élément de fixation, dans laquelle la zone de fixation comporte un évidement interne (41) situé dans une face interne (31, 131) de la première partie latérale ou une face interne de la deuxième partie latérale, l'évidement interne étant adjacent à la première extrémité latérale (22, 122) ou à la deuxième extrémité latérale (24, 124) de la partie frontale, le porte dosimètre comportant une embase de fixation (50) qui est fixée dans l'évidement interne par l'élément de fixation.

Grâce à ces caractéristiques, il est possible d'intégrer un dosimètre dans la monture et, de part la localisation du porte dosimètre et donc du dosimètre qui est situé proche d'un œil de l'utilisateur, de mesurer avec précision la dose reçue par les yeux de l'utilisateur car le dosimètre se situe au plus près de l'œil de l'utilisateur. La mesure de la dose de rayonnements reçue par les yeux de l'utilisateur par la monture portant un dosimètre permet notamment de surveiller l'exposition individuelle et de s'assurer que les agents restent en dessous des limites d'exposition réglementaires établies pour protéger leur santé. Cela permet de prendre des mesures correctives si nécessaire pour minimiser l'exposition future. En d'autres termes, il est possible d'adapter le travail de l'agent et de diminuer le risque de pathologie qui sont développées à la suite d'une exposition aux rayonnements ionisants, tel que la cataractes qui correspond à une opacité du cristallin.

Selon des modes de réalisation, une telle monture peut comporter une ou plusieurs des caractéristiques suivantes.

Selon un mode de réalisation, la chambre de réception est située en vis-à-vis d'une zone périphérique du premier logement ou une zone périphérique du deuxième logement. Selon un mode de réalisation, la zone périphérique présentant la forme d'une bande d'une largeur inférieure à 2 cm, préférentiellement d'une largeur inférieure à 1,5 cm ou 1 cm, et encore plus préférentiellement d'une largeur inférieure à 0,5 cm, et non nulle.

Ainsi, le porte dosimètre n'entrave pas la vision de l'agent portant la monture et permet, lorsqu'un dosimètre est inséré dans le porte dosimètre une mesure précise de la dose de rayonnements ionisants reçue par l'œil de l'agent.

Selon un mode de réalisation, le porte dosimètre est saillant depuis une partie supérieure de la zone d'angle.

Selon un mode de réalisation, le porte dosimètre est saillant depuis une partie inférieure de la zone d'angle.

Selon un mode de réalisation, le porte dosimètre est monobloc. Dans ce cas, le porte dosimètre est par exemple réalisé par impression 3D.

Selon un mode de réalisation, la partie frontale, le porte dosimètre et une portion de la première partie latérale ou de la deuxième partie latérale portant ledit porte dosimètre sont monobloc. Ainsi, la fabrication de la monture est accélérée, facilitée et peut être réalisée par impression 3D. Comme le porte dosimètre est fixé de manière amovible dans la monture, le porte dosimètre peut être remplacé ce qui facilite l'entretien et la réparation de la monture. En outre, l'amovibilité du porte dosimètre permet d'adapter les dimensions du porte dosimètre et particulièrement de la chambre de réception à une pluralité de dosimètres présentant des dimensions différentes. Ainsi, la monture s'adapte parfaitement au besoin de mesure de doses de l'agent.

Selon un mode de réalisation, l'évidement interne est situé au niveau d'une zone d'angle formée par l'intersection d'une surface interne de la partie frontale et d'une surface interne de la première partie latérale ou de la deuxième partie latérale.

Selon un mode de réalisation, l'évidement interne est situé au niveau d'une partie supérieure de la zone d'angle.

Selon un mode de réalisation, l'évidement interne est situé au niveau d'une partie inférieure de la zone d'angle.

Selon un mode de réalisation, l'évidement interne est situé au niveau d'une partie centrale de la zone d'angle. La partie centrale étant située entre la partie inférieure et la partie supérieure de la zone d'angle.

Selon un mode de réalisation, l'évidement interne présente une forme rectangulaire.

Selon un mode de réalisation, l'évidement interne présente une profondeur correspondant à au moins 50% de l'épaisseur de la première partie latérale ou de la deuxième partie latérale, préférentiellement au moins 60 % ou 70%, plus préférentiellement au moins 80%, par exemple au moins 90%, 95 %, 98% ou 99%. Selon un mode de réalisation, l'évidement interne présente une profondeur inférieure à 100% de l'épaisseur de la première partie latérale ou de la deuxième partie latérale.

Selon un mode de réalisation, l'élément de fixation est choisi parmi une vis ou une couche de colle.

Selon un mode de réalisation, l'embase de fixation est fixée dans l'évidement interne via une couche de colle qui est située dans le fond de l'évidement interne, entre l'embase de fixation et le fond de l'évidement interne.

Selon un mode de réalisation, l'embase de fixation est fixée dans l'évidement interne via au moins une vis, la vis traversant le fond de l'évidement interne depuis une surface externe de la première partie latérale ou de la deuxième partie latérale et étant fixé dans l'embase de fixation.

Selon un mode de réalisation l'embase de fixation comporte un orifice destiné à recevoir une vis. Selon un mode de réalisation préféré, l'embase de fixation comporte un deuxième orifice destiné à recevoir une vis.

Selon un mode de réalisation préféré, l'embase de fixation est fixée dans l'évidement interne par au moins une vis, préférentiellement par deux vis.

Selon un mode de réalisation, l'orifice destiné à recevoir une vis ne débouche pas dans la chambre de réception.

Selon un mode de réalisation, le fond de l'évidement interne comprend au moins un orifice traversant qui traverse la première partie latérale ou la deuxième partie latérale, l'orifice traversant étant destiné à être traversé par une vis. Selon un mode de réalisation préféré, le fond de l'évidement interne comprend deux orifices traversants.

Selon un mode de réalisation, la chambre de réception comporte une ouverture supérieure destinée à insérer le dosimètre. Selon un mode de réalisation, la chambre de réception comporte en outre un orifice d'extraction inférieur destiné à faciliter l'extraction du dosimètre, l'orifice d'extraction inférieur présentant des dimensions inférieures aux dimensions du dosimètre afin d'empêcher que le dosimètre traverse l'orifice d'extraction et afin de retenir le dosimètre dans la chambre de réception.

Selon un mode de réalisation, la chambre de réception comporte deux parois latérales destinées à maintenir le dosimètre dans la chambre de réception par pincement du dosimètre. Dans ce mode de réalisation, le dosimètre est par exemple inséré en exerçant une légère force de pression pour l'insérer le dosimètre dans la chambre de réception.

Selon un mode de réalisation, les deux parois latérales sont situées en vis-à-vis l'une de l'autre.

Selon un mode de réalisation, les deux parois latérales comportent une paroi latérale interne et une paroi latérale externe.

Selon un mode de réalisation, la paroi latérale externe et/ou interne est pleine. En d'autres termes, la paroi latérale externe et/ou interne ne comprend pas d'ouverture centrale.

Selon un mode de réalisation, la paroi latérale externe est opaque afin de protéger le dosimètre des rayons lumineux pouvant perturber la mesure de la dose spécifique par le dosimètre.

Selon un mode de réalisation, la paroi latérale interne est opaque.

Selon un mode de réalisation, les deux parois latérales sont opaques.

Ainsi, le dosimètre situé dans le porte dosimètre aura une faible exposition aux rayons lumineux. Une telle monture permet donc d'obtenir une mesure plus précise par le dosimètre, mesure qui ne sera pas altérée par les rayons lumineux.

Selon un mode de réalisation, la paroi latérale interne et/ou la paroi latérales externe du porte dosimètre est réalisée en un matériau choisi parmi : les résines, les plastiques et les polyamides tel que le Grilamid^{®}.

Selon un mode de réalisation, la chambre de réception comporte une ouverture centrale destinée à former un jour pour le dosimètre, l'ouverture centrale étant située en vis-à-vis du premier logement ou du deuxième logement, en particulier en vis-à-vis de la zone périphérique du premier ou deuxième logement.

Selon un mode de réalisation l'ouverture centrale présente des dimensions plus petites que les dimensions du dosimètre afin que ledit dosimètre ne puisse pas traverser l'orifice central.

Selon un mode de réalisation, l'ouverture centrale est circulaire.

Selon un mode de réalisation, la chambre de réception est située en vis-à-vis d'une zone périphérique du premier ou deuxième logement afin de ne pas entraver la vision de l'utilisateur portant la monture.

Selon un mode de réalisation, la chambre de réception est destinée à être située en vis-à-vis du canthus externe ou du canthus interne de l'œil de l'utilisateur.

Selon un mode de réalisation, le porte dosimètre est un premier porte dosimètre, la monture comprend en outre un deuxième porte dosimètre, le deuxième porte dosimètre comportant une deuxième chambre de réception destinée à recevoir un deuxième dosimètre, le deuxième porte dosimètre étant saillant derrière la partie frontale dans le prolongement du premier logement ou du deuxième logement, de sorte que la chambre de réception et le deuxième dosimètre soient situés entre celui parmi le premier logement et le deuxième logement qui ne porte pas le premier porte dosimètre et les yeux de l'utilisateur.

Ainsi, il est possible d'intégrer deux dosimètres dans la monture. Il est donc possible de mesurer avec précision la dose reçue par les deux yeux de l'agent portant la monture avec deux dosimètres identiques ou différents. Il est possible de mesurer par exemple deux types de rayonnements ionisants avec deux dosimètres différents.

Selon des modes de réalisations, le deuxième porte dosimètre peut présenter les mêmes caractéristiques que celles indiquées pour le premier porte dosimètre.

Selon un mode de réalisation, la zone de fixation pour porte dosimètre est une première zone de fixation, la première partie latérale ou la deuxième partie latérale de la monture comprend en outre une deuxième zone de fixation pour porte dosimètre, la deuxième zone de fixation comporte un deuxième évidement interne situé dans une face interne de celle parmi la première partie latérale et la deuxième partie latérale qui ne comporte pas la première zone de fixation, le deuxième évidement interne étant adjacent à celle parmi la première extrémité latérale et la deuxième extrémité latérale de la partie frontale qui ne comporte pas la première zone de fixation.

Selon des modes de réalisation, la deuxième zone de fixation pour porte dosimètre comprend une ou plusieurs des caractéristiques précitées pour la première zone de fixation.

Selon un mode de réalisation, la première zone de fixation et la deuxième zone de fixation comprennent les mêmes caractéristiques.

Selon un mode de réalisation, la première zone de fixation et la deuxième zone de fixation sont différentes l'une de l'autre.

Selon un mode de réalisation, la première zone de fixation et la deuxième zone de fixation sont située à l'opposée l'une de l'autre.

Selon un mode de réalisation, le deuxième évidement interne est comblé par un bouchon amovible. Ainsi, cela empêche la saleté, l'humidité ou d'autres contaminants de pénétrer dans l'évidement.

Selon un mode de réalisation, le bouchon est fixé par un élément de fixation.

Selon un mode de réalisation, le bouchon est fixé par le même élément de fixation que l'élément de fixation fixant le porte dosimètre à la zone de fixation.

Selon un mode de réalisation, le bouchon comble entièrement l'évidement interne.

Selon un mode de réalisation, le bouchon comporte un orifice destiné à recevoir une vis. Selon un mode de réalisation préféré, le bouchon comporte un deuxième orifice destiné à recevoir une vis.

Selon un mode de réalisation, le bouchon est fixé dans l'évidement interne par au moins une vis, préférentiellement deux vis.

Selon un mode de réalisation, l'orifice destiné à recevoir une vis ne traverse pas le bouchon de part en part.

Selon un mode de réalisation, le bouchon est monobloc.

Selon un mode de réalisation, une pièce de pont destinée à retenir les premier et deuxième verres minéraux est fixée sur une face externe de la zone de pont, dans laquelle la pièce de pont comporte une portion de retenue qui déborde vers les premier et deuxième logements pour empêcher que les premier et deuxième verres minéraux se délogent.

Ainsi, la sécurité de la monture est renforcée. En effet, en cas de torsion s'exerçant par exemple sur l'arrière de la monture, un tel arrangement permet de maintenir le premier verre minéral dans le premier drageoir et le deuxième verre minéral dans le deuxième drageoir, afin d'empêcher qu'ils soient éjectés de la monture.

Selon un mode de réalisation, la pièce de pont présente la forme d'une platine.

Selon un mode de réalisation, la pièce de pont est réalisée en un matériau choisi parmi : les résines, les plastiques et les polyamides tel que le Grilamid^{®}.

Selon un mode de réalisation, la pièce de pont présente une épaisseur comprise entre 0,5 et 2 mm, préférentiellement 1 mm.

Selon un mode de réalisation, la portion de retenue de la pièce de pont déborde d'une distance comprise entre 0,05 et 0,15 mm vers les premier et deuxième logements, préférentiellement de 0,1 mm.

Selon un mode de réalisation, le premier logement comporte premier drageoir et le deuxième logement comporte un deuxième drageoir, dans laquelle le premier logement comprend un premier verre minéral qui est logé dans le premier drageoir et dans laquelle le deuxième logement comprend un deuxième verre minéral qui est logé dans le deuxième drageoir, le premier et le deuxième verres minéraux comportant une deuxième charge capable d'absorber des rayonnements ionisants.

Selon un mode de réalisation, la première charge et la deuxième charge capable d'absorber des rayonnements ionisants comprennent des particules d'au moins un matériau choisi parmi : le plomb, les terres rares, le bismuth, l'antimoine, l'étain, le tungstène, le baryum, le tantale, les alliages et les oxydes de ceux-ci. Selon un mode de réalisation préféré, la première charge et la deuxième charge comprennent des particules de plomb.

Selon un mode de réalisation, la monture pour lunettes permet une protection contre les rayonnements ionisants choisis parmi les rayonnements électromagnétiques de type X ou de type gamma.

Selon un mode de réalisation, la première charge est située sur ou dans : la partie frontale, la première partie latérale et/ou la deuxième partie latérale. Préférentiellement, la première charge est située au moins dans la partie frontale.

Selon un mode de réalisation, la deuxième charge est située sur ou dans le premier et le deuxième verres minéraux.

Selon un mode de réalisation, le premier logement comporte un troisième drageoir espacé du premier drageoir et le deuxième logement comporte un quatrième drageoir espacé du deuxième drageoir, dans laquelle le premier logement comprend un troisième verre qui est logé dans le troisième drageoir et le deuxième logement comprend un quatrième verre qui est logé dans le quatrième drageoir, les troisième et quatrième verres étant positionnés de manière à être situés entre les premier et deuxième verres et les yeux d'un utilisateur.

Selon un mode de réalisation, le troisième drageoir et le quatrième drageoir sont respectivement espacés du premier drageoir et du deuxième drageoir d'une distance comprise entre 1 et 4 mm.

Selon un mode de réalisation, l'espacement située entre le premier verre minéral et le troisième verre forme un premier espace interne et dans laquelle l'espacement située entre le deuxième verre minéral et le quatrième verre forme un deuxième espace interne, dans laquelle les premier et deuxième espaces internes sont hermétiques.

Ainsi, les caractéristiques de la monture empêchent la saleté, l'humidité ou d'autres contaminants de pénétrer dans le premier espace interne ou le deuxième espace interne afin de préserver la clarté visuelle pour l'agent.

Selon un mode de réalisation, un joint étanche est positionné sur le pourtour des premier, deuxième, troisième et quatrième drageoirs afin d'obtenir un espace interne et un deuxième espace interne hermétiques.

Selon un mode de réalisation, le troisième et le quatrième verres sont fabriqués à partir de matière plastique transparente, préférentiellement à partir d'un polymère thermoplastique.

Selon un mode de réalisation, le troisième et le quatrième verres sont fabriqués à partir de polycarbonate.

Selon un mode de réalisation, le troisième verre et/ou le quatrième verre présente une correction visuelle.

Grâce à ces caractéristiques, la correction visuelle est facile à appliquer. Ainsi, il n'est pas nécessaire de réaliser une correction visuelle sur des verres minéraux complexes et cassants. Les coûts de production des verres minéraux de protection contre les rayonnements ionisants peuvent donc être réduits, en comparaison avec le coût d'une correction visuelle qui serait appliquée sur des verres minéraux.

Selon un mode de réalisation, le troisième verre et/ou le quatrième verre corrige un défaut visuel choisi parmi : la myopie, l'hypermétropie, l'astigmatisme isolé ou associé à une myopie ou une hypermétropie, ou bien une presbytie isolée ou associée à une myopie, une hypermétropie et/ou un astigmatisme. Les troisième et quatrième verres peuvent être à simple foyer, à double foyer ou progressifs.

Selon un mode de réalisation, la première partie latérale est une première branche de monture et la deuxième partie latérale est une deuxième branche de monture, lesdites première et deuxième branches étant rattachées à la première partie frontale via respectivement une première articulation et une deuxième articulation, la première articulation et la deuxième articulation comprenant une rotule.

Ainsi, la monture est davantage ergonomique. En outre, grâce à ces caractéristiques, l'articulation comprenant une rotule permet de reprendre les efforts de manipulation de la monture et ainsi permet notamment de protéger les premier et deuxième verres minéraux des torsions mécaniques qui pourraient les endommager.

Selon un mode de réalisation, la première articulation et la deuxième articulation comprennent un rappel élastique pour maintenir la première branche et la deuxième branche, en l'absence de contraintes externes, dans une position d'utilisation afin que l'agent puisse porter la monture normalement.

Selon un mode de réalisation, les branches de la monture présentent une longueur d'au moins 5 cm, par exemple entre 5 et 15 cm.

Selon un mode de réalisation, la chambre de réception comporte un dosimètre destiné à mesurer une dose de rayonnements ionisants, par exemple de rayons X, gamma, bêta ou neutrons, reçue par un agent.

Selon un mode de réalisation, le premier porte dosimètre porte un dosimètre destiné à mesurer une dose de rayons gamma reçu par un agent au niveau des yeux et le deuxième porte dosimètre porte un dosimètre destiné à mesurer les neutrons reçus par un agent au niveau des yeux.

Selon un mode de réalisation, le dosimètre est un dosimètre passif ou dosimètre à lecture différée. Selon un mode de réalisation, le dosimètre présente la forme d'un cylindre aplati. Le dosimètre présente par exemple une forme cylindrique circulaire aplatie, c'est-à-dire avec une épaisseur relativement faible par rapport à son diamètre.

Selon un mode de réalisation, la monture pour lunettes de protection contre les rayonnements ionisants est utilisée dans le domaine de la médecine, notamment, en radiologie diagnostique, en radiologie interventionnelle, en radiothérapie ou en curiethérapie ou en médecine nucléaire, par le personnel soignant et/ou par le personnel soigné.

Selon un mode de réalisation, la monture pour lunettes de protection contre les rayonnements ionisants est utilisée dans le domaine de l'énergie nucléaire civile ou militaire, notamment pour les activités minières ou pour les traitements et la fabrication des combustibles nucléaires.

Selon des modes de réalisation, la zone de fixation comprend une ou plusieurs des caractéristiques précitées.

Selon un mode de réalisation, l'évidement interne est comblé par un premier bouchon amovible.

Selon un mode de réalisation, la zone de fixation pour porte dosimètre est une première zone de fixation, la monture comprend en outre une deuxième zone de fixation pour porte dosimètre, la deuxième zone de fixation comporte un deuxième évidement interne situé dans une face interne de celle parmi la première partie latérale et la deuxième partie latérale qui ne comporte pas la première zone de fixation, le deuxième évidement interne étant situé au niveau d'une deuxième zone d'angle formée par l'intersection de la surface interne de la partie frontale et de la surface interne de celle parmi la première partie latérale et la deuxième partie latérale qui ne comporte pas la première zone de fixation, le deuxième évidement interne étant destiné à recevoir un deuxième porte dosimètre comportant une deuxième chambre de réception destinée à recevoir un deuxième dosimètre et une deuxième embase de fixation destinée à être fixée dans le deuxième évidement interne, le deuxième porte dosimètre étant destiné à faire saillie derrière la partie frontale dans le prolongement du premier logement ou du deuxième logement, de sorte que la chambre de réception et le dosimètre soient situés entre celui parmi le premier logement et le deuxième logement qui ne porte pas le premier porte dosimètre et les yeux de l'utilisateur.

Selon des modes de réalisation, la deuxième zone de fixation comprend une ou plusieurs des caractéristiques précitées.

Selon un mode de réalisation, le deuxième évidement interne est comblé par un deuxième bouchon amovible.

Selon des modes de réalisation, le premier et/ou le deuxième bouchon amovible comporte les caractéristiques du bouchon précité.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
La figure 1 représente une vue de face d'une monture pour lunette de protection, selon un premier mode de réalisation.
La figure 2 représente une vue en perspective éclaté de la monture illustrée sur la figure 1.
La figure 3 représente une vue en perspective d'une monture pour lunette selon un deuxième mode de réalisation.
La figure 4 représente une vue en perspective d'un porte dosimètre adapté pour être intégré dans la monture selon le deuxième mode de réalisation.
La figure 5 représente une vue du dessus du porte dosimètre illustré sur la figure 4.
La figure 6 représente une vue en perspective d'un bouchon amovible adapté pour être intégré dans la monture selon le deuxième mode de réalisation.

### Description des modes de réalisation

Par convention, les termes « interne », « externe », « supérieur » et « inférieur » sont utilisés pour définir la position relative d'un élément en référence à la monture lorsqu'elle est portée par un agent. Le terme « interne » signifie donc un élément ou une zone qui est situé vers l'intérieur, c'est-à-dire vers le visage de l'agent par opposition au terme « externe » qui renvoie à un élément ou une zone qui est situé vers l'extérieur. Le terme « supérieur » signifie un élément ou une zone située vers le haut de la monture et le terme « inférieur » signifie un élément ou une zone située vers le bas de la monture.

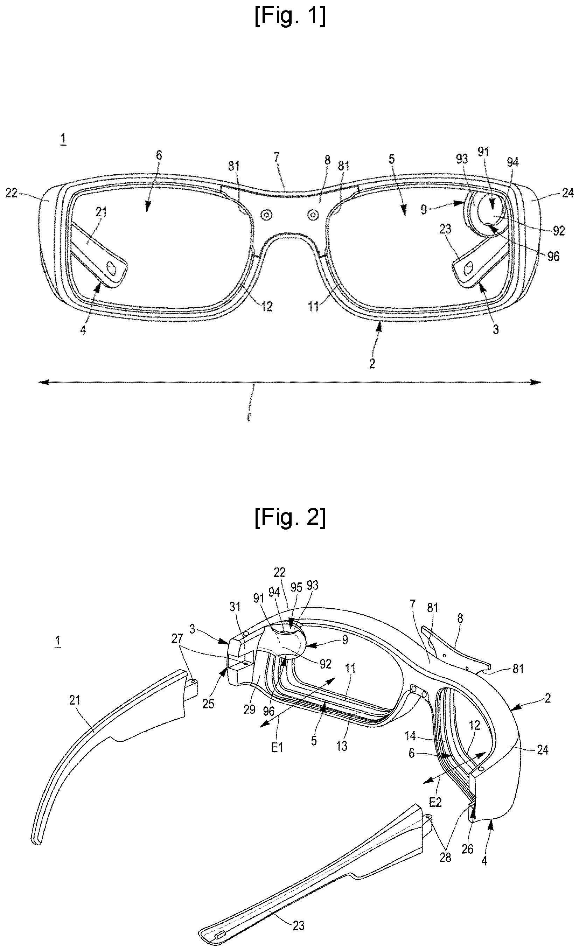

Les figures 1 et 2 illustrent une monture 1 pour lunettes protection contre les rayonnements ionisants.

La monture 1 comprend une partie frontale 2 et deux parties latérales 3, 4.

La première partie latérale 3 s'étend depuis une première extrémité latérale 22 de la partie frontale 2 et la deuxième partie latérale 4 s'étend depuis une deuxième extrémité latérale 24 de la partie frontale 2.

La première partie latérale 3 s'étend depuis une première extrémité latérale 22 de la partie frontale 2 et comprend une première branche de monture 21.

La deuxième partie latérale 4 s'étend depuis une deuxième extrémité latérale 24 de la partie frontale 2 et comprend une deuxième branche de monture 23.

La première et la deuxième branches de monture 21 et 23 présentent une forme adaptée pour être correctement positionnée sur les oreilles de l'utilisateur, par exemple via une courbure présentant une concavité.

Les première et deuxième branches de monture 21 et 23 sont fixées respectivement à une première et à une deuxième zones extrémités latérales 25, 26 de la première et deuxième parties latérales 3, 4 par une première articulation charnières 27 et une deuxième articulation charnière 28. Les première et deuxième articulations charnières 27, 28 autorisent un premier mouvement de rotation permettant de plier vers l'intérieur la première et la deuxième branches de monture 21 et 23, et autorise un deuxième mouvement permettant de repositionner les première et deuxième branches 21 et 23 de monture dans une position d'utilisation. Les première et deuxième articulations charnières 27, 28 bloquent les autres possibilités de mouvement des branches, par exemple un mouvement de rotation vers l'extérieur, c'est-à-dire un mouvement opposé au premier mouvement, lorsque les première est deuxième branches 21 et 23 sont dans la position d'utilisation.

Dans une variante non représentée, l'articulation de la fixation des première et deuxième branches 21 et 23 sont des articulations comprenant chacune une rotule équipée d'un élément de rappel élastique. Cette articulation autorise davantage de mouvements de rotation de chacune des première et deuxième branches 21 et 23 par rapport aux zones extrémités latérales 25, 26 illustrées sur la figure 2.

La partie frontale 2 de la monture 1 présente en outre un premier logement 5 et un deuxième logement 6 espacé du premier logement 5 par une zone de pont 7.

Le premier logement 5 comporte un premier drageoir 11 formé dans l'épaisseur de la partie frontale 2 au niveau du premier logement 5, le premier drageoir 11 présentant la forme d'une rainure s'étendant sur le contour du premier logement 5. Le premier drageoir 11 est destiné à loger et à maintenir un premier verre minéral (non représenté) dans le premier logement 5.

De manière analogue, le deuxième logement 6 comporte un deuxième drageoir 12 formé dans l'épaisseur de la partie frontale 2 au niveau du deuxième logement 6, le deuxième drageoir 12 présentant la forme d'une rainure s'étendant sur le contour du deuxième logement 6. Le deuxième drageoir 12 est destiné à loger et à maintenir un deuxième verre minéral (non représenté) dans le deuxième logement 6.

Les premier et deuxième verres minéraux sont réalisés dans une matière minérale transparente qui comporte une deuxième charge permettant notamment l'absorption des rayonnements X et gamma, par exemple une charge de plomb.

Le premier logement 5 comporte en outre un troisième drageoir 13 espacé du premier drageoir 11 selon la direction d'épaisseur E1 de la partie frontale 2 et le deuxième logement 6 comporte un quatrième drageoir 14 espacé du deuxième drageoir 12 selon la direction d'épaisseur E2 de la partie frontale 2.

Le troisième drageoir est destiné à loger et à maintenir un troisième verre (non représenté) dans le premier logement 5 et le quatrième drageoir 14 est destiné à loger et à maintenir un quatrième verre (non représenté) dans le deuxième logement 6.

Le troisième et quatrième verres sont par exemple en polycarbonate, comprenant optionnellement une correction visuelle.

Ainsi, cela permet d'obtenir une monture avec deux paires de verres, le troisième et quatrième verres étant destinés à être plus proche des yeux de l'utilisateur et à protéger les yeux de l'agent d'une éventuelle casse des verres minéraux qui sont fragiles et cassants.

La partie frontale 2 de la monture 1 porte en outre une pièce de pont 8 fixée sur une face externe de la zone de pont 7 avec par exemple des vis. La pièce de pont 8 présente une portion de retenue 81 faisant légèrement saillie, selon la direction de largeur l de la monture 1 devant chacun des premier et deuxième logements 5, 6 afin de retenir les premier et deuxième verres minéraux (non représentés) dans les premier et deuxième drageoirs 11 et 12, sans obturer la vision de l'agent portant la monture 1.

La monture 1 comprend en outre un porte dosimètre 9 situé au niveau d'une zone supérieure d'angle formée par l'intersection d'une surface interne 31 de la zone extrémité latérale 25 et d'une surface interne 29 de la partie frontale 2, au niveau de l'extrémité latérale 22 de la partie frontale 2.

Le porte dosimètre 9 fait saillie depuis la zone d'extrémité latérale 25 vers le premier logement 5 afin d'être situé entre le premier logement et les yeux de l'agent lorsqu'il porte la monture 1, sans gêner la vision de l'agent portant la monture 1. Ainsi, le porte dosimètre 9 est légèrement visible par l'agent au niveau de la périphérie externe du champ de vision et ne gène donc pas l'agent pour effectuer ses tâches.

Le porte dosimètre 9 comporte une chambre de réception 91 globalement circulaire destinée à réceptionner un dosimètre (non représenté). La chambre de réception 91 comporte une paroi interne 92 pleine espacée d'une paroi externe 93 située en vis-à-vis de la paroi interne 92 et une ouverture supérieure 95.

Les dimensions de l'ouverture supérieure 95 sont suffisantes pour laisser passer un dosimètre (non représenté) et l'espacement entre la paroi interne 92 et la paroi externe 93 est suffisant pour y loger le dosimètre (non représenté).

La paroi externe 93 comporte une ouverture circulaire 94 ménagée au niveau du centre de la paroi externe 93 afin de laisser un jour pour le dosimètre. L'ouverture circulaire présente un diamètre inférieur au diamètre du dosimètre afin d'empêcher que le dosimètre passe à travers l'ouverture circulaire 94.

Ainsi, lorsque le dosimètre est logé dans la chambre de réception 91, il sera apte à mesurer avec précision la dose de rayonnements ionisants effectivement reçu par l'œil de l'agent portant la monture 1 avec une précision importante. En effet, grâce au porte dosimètre 9, l'emplacement du dosimètre sera au plus proche de l'oeil de l'utilisateur et derrière les éventuels verres positionnés dans le premier et troisième drageoirs 11, 13. Le dosimètre recevra donc la même quantité de rayonnements ionisants que la quantité effectivement reçue par les yeux de l'agent.

La chambre de réception 91 comporte en outre un orifice d'extraction inférieur 96 qui permet de faciliter l'extraction du dosimètre de la chambre de réception 94. L'orifice d'extraction inférieur 96 débouche dans la chambre de réception 91 et présente des dimensions inférieures aux dimensions du dosimètre afin d'empêcher que le dosimètre sorte involontairement de la chambre de réception 91 en passant à travers l'orifice d'extraction inférieur 96.

Le dosimètre peut être délogé de la chambre de réception 91 en insérant un outil d'extraction tel qu'une tige dans l'orifice d'extraction afin de pousser le dosimètre à travers l'ouverture supérieure 95 pour l'extraire du porte dosimètre 9.

L'orifice d'extraction 96 comporte par exemple une encoche ménagée sur la paroi interne 92 afin de permettre d'extraire facilement le dosimètre du logement avec l'outil d'extraction ou un doigt d'un agent.

La monture 1 est par exemple réalisée en une matière plastique qui comporte une première charge permettant notamment l'absorption des rayonnements X et gamma, par exemple une charge de plomb.

La partie frontale 2, la première zone d'extrémités latérale 25 de la première partie latérale 3, la deuxième zone d'extrémité latérale 26 de la deuxième partie latérale 4 et le porte dosimètre 9 de la monture 1 peuvent être réalisés de manière monobloc, par exemple par impression 3D.

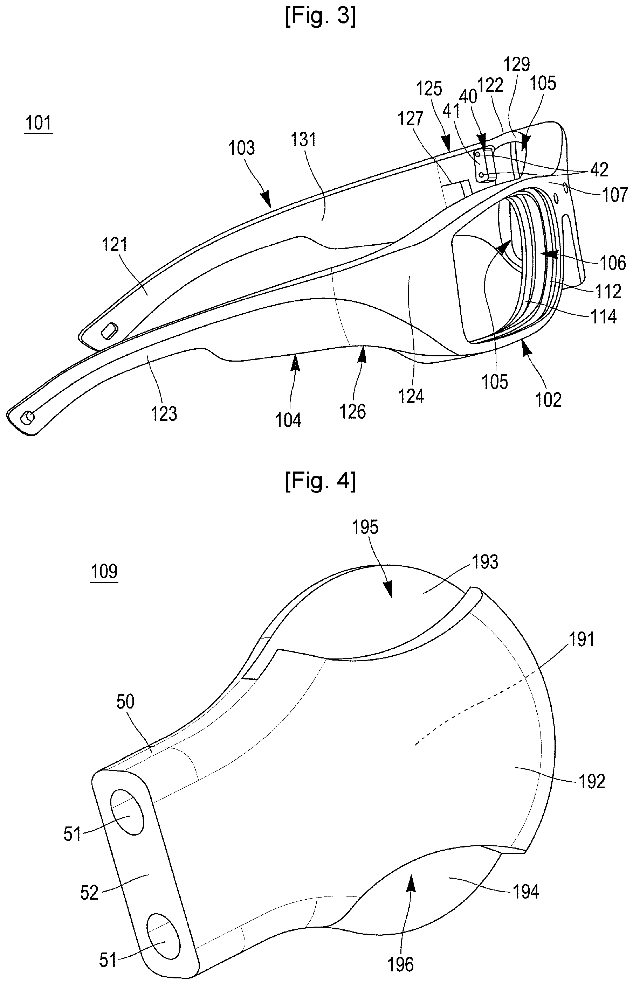

Il est décrit avec les figures 3 à 5 un autre mode de réalisation de la monture.

Les éléments identiques ou similaires portent le même chiffre de référence, incrémenté de 100 par rapport aux numéros de références indiqués sur les figures 1 et 2.

La monture 101 illustrée sur la figure 3 diffère de la monture 1 illustrée avec les figures 1 et 2 en ce que le porte dosimètre 109 est un élément amovible de la monture 109 et que le porte dosimètre ne présente pas d'ouverture circulaire ménagée au niveau du centre de la paroi externe.

La monture 101 comprend une zone de fixation 40 située au niveau supérieur de l'angle formé par l'intersection d'une surface interne 131 de la zone extrémité latérale 125 et d'une surface interne 129 de la partie frontale 102, au niveau de l'extrémité latérale 122 de la partie frontale 102.

La zone de fixation 40 comporte un évidement interne 41 formant un creux globalement rectangulaire avec un fond présentant deux orifices traversants 42 pour fixer un porte dosimètre 109 tel que présenté avec la figure 4.

Le porte dosimètre 109 diffère du porte dosimètre 9 illustré avec les figures 1 et 2 en ce qu'il comporte une embase de fixation 50 au niveau d'une extrémité latérale du porte dosimètre 109.

L'embase de fixation 50 présente deux orifices 51 situés au niveau d'une surface latérale 52 qui sont destinés à coopérer avec les deux orifices traversants 42 de l'évidement interne 41.

L'embase de fixation 50 est dimensionnée pour remplir complètement l'évidement interne 41.

Afin d'intégrer le porte dosimètre 109 à la monture 101, il convient d'insérer l'embase de fixation 50 dans l'évidement interne 41 jusqu'au fond de l'évidement interne 41 afin que les orifices traversants 42 soient alignés et débouchent en face des orifices 51 afin de permettre le passage d'un élément de fixation (non représenté) tel qu'une vis de fixation par orifices traversants 42.

Ainsi, il est possible de retirer et de changer le porte dosimètre 109 de la monture 101 au besoin. Par exemple, le porte dosimètre 109 peut être remplacé par un autre porte dosimètre comprenant une embase de fixation identique à l'embase de fixation 50 et une chambre de réception présentant des dimensions différentes des dimensions de la chambre de réception du porte dosimètre 109 afin de s'adapter à un autre dosimètre.

En outre, la paroi externe 193 est une paroi opaque, c'est-à-dire une paroi ne comportant pas d'ouverture ménagée dans la paroi externe 193. Ainsi, le dosimètre installé dans le porte dosimètre aura une faible exposition aux rayons lumineux ce qui permet d'augmenter la fiabilité et la précision de la mesure de la dose de rayonnements ionisants par le dosimètre.

Dans une variante de réalisation non représentée, la monture 101 comporte une deuxième zone de fixation similaire à la zone de fixation 40. Cette deuxième zone de fixation est située à opposer de la première zone de fixation, c'est-à-dire située au niveau supérieur de l'angle formé par l'intersection d'une surface interne de la zone extrémité latérale 126 et d'une surface interne 129 de la partie frontale 102, au niveau de l'extrémité latérale 124 de la partie frontale 102. Ainsi, selon cette variante de réalisation, la monture 101 peut être adaptée et comporter :
i) un unique porte dosimètre 109 située dans l'une des première ou deuxième zones de fixations, ou
ii) un premier porte dosimètre 109 situé dans la première zone de fixation et un deuxième porte dosimètre situé dans la deuxième zone de fixation. Le deuxième porte dosimètre pouvant par exemple être identique, similaire ou différent du premier porte dosimètre 109, ou
iii) aucun porte dosimètre.

Dans le cas i) : un bouchon peut être avantageusement fixé dans l'évidement interne dans lequel le porte dosimètre n'est pas fixé afin de boucher l'évidement interne.
Dans le cas iii) : de manière similaire au cas i), un premier et un deuxième bouchons peuvent être fixés dans les premier et deuxième évidements internes afin de boucher les deux évidements internes.

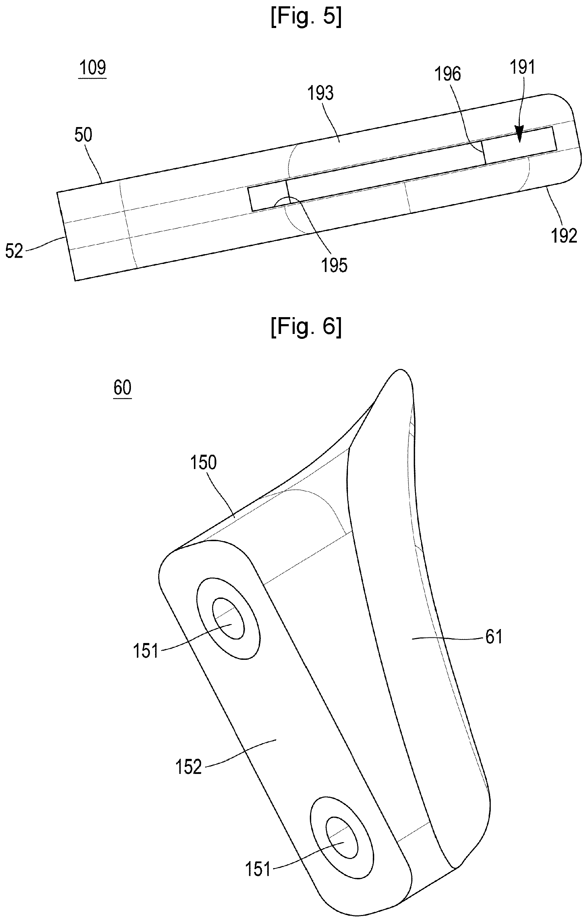

Un bouchon adapté pour boucher un évidement interne est illustré avec la figure 6.

Le bouchon 60 présente des dimensions adaptées pour combler entièrement par exemple l'évidement interne 41 illustré sur la figure 3. Le bouchon 60 comprend une embase de fixation 150 au niveau d'une extrémité latérale 152 du bouchon 60.

L'embase de fixation 150 présente des caractéristiques similaires à l'embase de fixation 50 du porte dosimètre 109, à savoir deux orifices 151 situés au niveau d'une surface latérale 152 qui sont destinés à coopérer avec les deux orifices traversants 42 de l'évidement interne 41.

L'extrémité latérale interne 61 du bouchon 60 présente en outre une courbure apte à établir une continuité de la surface interne 131 de la zone extrémité latérale 125 ou de la surface interne de la zone extrémité latérale 126. En d'autres termes, lorsque le bouchon 60 est installé, il ne forme pas de protubérance ou de creux par rapport à la surface interne dans laquelle le bouchon 60 est installé.

L'installation du bouchon 60 permet notamment d'obtenir des montures ergonomiques agréable à porter par l'agent.

En outre, le bouchon 60 comble totalement l'évidement ce qui permet de limiter l'encrassement de l'évidement interne bouché par le bouchon 60.

Afin d'intégrer le bouchon 60 à la monture 101, il convient d'insérer l'embase de fixation 150 du bouchon 60 dans l'évidement 41 jusqu'au fond de l'évidement 41 afin que les orifices traversants 42 soient alignés et débouchent dans les orifices 151 afin de permettre le passage d'un élément de fixation (non représenté) tel qu'une vis de fixation par orifices traversants.

Les éléments de fixations utilisés pour fixer le bouchon peuvent être les mêmes que les éléments de fixation pour fixer le porte dosimètre 109.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Monture pour lunettes de protection contre les rayonnements ionisants, la monture (1, 101) comprenant une partie frontale (2, 102), une première partie latérale (3, 103) et une deuxième partie latérale (4, 104) s'étendant respectivement depuis une première et une deuxième extrémités latérales (25, 26, 125, 126) de la partie frontale,
dans laquelle la monture comprend une première charge capable d'absorber des rayonnements ionisants,
dans laquelle la partie frontale comporte un premier logement (5, 105) qui est destiné à porter un premier verre minéral et un deuxième logement (6, 106) qui est destiné à porter un deuxième verre minéral, le premier et le deuxième logements étant espacés l'un de l'autre par une zone de pont (7, 107) de la partie frontale,
dans laquelle la monture comprend un porte dosimètre (9, 109), le porte dosimètre comportant une chambre de réception (91, 191) destinée à recevoir un dosimètre, le porte dosimètre étant saillant derrière la partie frontale dans le prolongement du premier logement ou du deuxième logement, de sorte que la chambre de réception et le dosimètre soient situés entre le premier logement ou le deuxième logement et les yeux de l'utilisateur,
dans laquelle la première partie latérale ou la deuxième partie latérale comporte une zone de fixation (40) pour porte dosimètre,
**caractérisée en ce que**
le porte dosimètre est saillant depuis la première partie latérale ou depuis la deuxième partie latérale depuis une zone d'angle formée par l'intersection d'une surface interne (29, 129) de la partie frontale et d'une surface interne (31, 131) de la première partie latérale ou de la deuxième partie latérale,
et le porte dosimètre est fixé à la zone de fixation de manière amovible par un élément de fixation,
et la zone de fixation comporte un évidement interne (41) situé dans une face interne (31, 131) de la première partie latérale ou une face interne de la deuxième partie latérale, l'évidement interne étant adjacent à la première extrémité latérale (22, 122) ou à la deuxième extrémité latérale (24, 124) de la partie frontale, le porte dosimètre comportant une embase de fixation (50) qui est fixée dans l'évidement interne par l'élément de fixation.

2. Monture selon la revendication 1, dans laquelle la chambre de réception comporte une ouverture supérieure (95, 195) destinée à insérer le dosimètre et un orifice d'extraction inférieur (96, 196) destiné à faciliter l'extraction du dosimètre, l'orifice d'extraction inférieur présentant des dimensions inférieures aux dimensions du dosimètre afin d'empêcher que le dosimètre traverse l'orifice d'extraction et afin de retenir le dosimètre dans la chambre de réception.

3. Monture selon l'une des revendications 1 à 2, dans laquelle la chambre de réception comporte deux parois latérales (92, 93, 192, 193) destinées à maintenir le dosimètre dans la chambre de réception par pincement du dosimètre.

4. Monture selon l'une des revendications 1 à 3, dans laquelle la chambre de réception comporte deux parois latérales (192, 193) opaques.

5. Monture selon l'une des revendications 1 à 4, dans laquelle la chambre de réception est située en vis-à-vis d'une zone périphérique du premier ou deuxième logement afin de ne pas entraver la vision de l'utilisateur portant la monture.

6. Monture selon l'une des revendications 1 à 5, dans laquelle le porte dosimètre est un premier porte dosimètre et dans laquelle la monture comprend un deuxième porte dosimètre, le deuxième porte dosimètre comportant une deuxième chambre de réception destinée à recevoir un deuxième dosimètre, le deuxième porte dosimètre étant saillant derrière la partie frontale dans le prolongement du premier logement ou du deuxième logement, de sorte que la chambre de réception et le deuxième dosimètre soient situés entre celui parmi le premier logement et le deuxième logement qui ne porte pas le premier porte dosimètre et les yeux de l'utilisateur.

7. Monture selon l'une des revendications 1 à 6, dans laquelle une pièce de pont (8) destinée à retenir les premier et deuxième verres minéraux est fixée sur une face externe de la zone de pont, dans laquelle la pièce de pont comporte une portion de retenue (81) qui déborde vers les premier et deuxième logements pour empêcher que les premier et deuxième verres minéraux se délogent.

8. Monture selon l'une des revendications 1 à 7, dans laquelle le premier logement comporte premier drageoir (11) et le deuxième logement comporte un deuxième drageoir (12, 112), dans laquelle le premier logement comprend un premier verre minéral qui est logé dans le premier drageoir et dans laquelle le deuxième logement comprend un deuxième verre minéral qui est logé dans le deuxième drageoir, le premier et le deuxième verres minéraux comportant une deuxième charge capable d'absorber des rayonnements ionisants.

9. Monture selon la revendication 8, dans laquelle le premier logement comporte un troisième drageoir (13) espacé du premier drageoir et le deuxième logement comporte un quatrième drageoir (14, 114) espacé du deuxième drageoir, dans laquelle le premier logement comprend un troisième verre qui est logé dans le troisième drageoir et le deuxième logement comprend un quatrième verre qui est logé dans le quatrième drageoir, les troisième et quatrième verres étant positionnés de manière à être situés entre les premier et deuxième verres et les yeux d'un utilisateur.

10. Monture selon la revendication 9, dans laquelle l'espacement située entre le premier verre minéral et le troisième verre forme un premier espace interne et dans laquelle l'espacement située entre le deuxième verre minéral et le quatrième verre forme un deuxième espace interne, dans laquelle les premier et deuxième espaces internes sont hermétiques.

11. Monture selon l'une des revendications 10 à 11, dans laquelle le troisième verre et/ou le quatrième verre présente une correction visuelle.

12. Monture selon l'une des revendications 1 à 11, dans laquelle la première partie latérale est une première branche de monture (21, 121) et la deuxième partie latérale est une deuxième branche de monture (23, 123), lesdites première et deuxième branches étant rattachées à la première partie frontale via respectivement une première articulation et une deuxième articulation,
la première articulation et la deuxième articulation comprenant une rotule.

## Patentansprüche

1. Brillengestell zum Schutz gegen ionisierende Strahlung, das Gestell (1, 101) umfassend ein Vorderteil (2, 102), ein erstes Seitenteil (3, 103) und ein zweites Seitenteil (4, 104), die sich jeweils von einem ersten und einem zweiten Ende (25, 26, 125) des Vorderteils erstrecken,
wobei das Brillengestell ein erstes Material umfasst, das zum Absorbieren von ionisierender Strahlung geeignet ist, wobei das Vorderteil eine erste Aufnahme (5, 105) zum Tragen eines ersten Mineralglases und eine zweite Aufnahme (6, 106) zum Tragen eines zweiten Mineralglases umfasst, wobei die erste und die zweite Aufnahme durch einen Stegbereich (7, 107) des Vorderteils voneinander beabstandet sind,
wobei das Gestell einen Dosimeterhalter (9, 109) mit einem Aufnahmeraum (91, 191) zur Aufnahme eines Dosimeters umfasst, wobei der Dosimeterhalter hinter dem Vorderteil in der Verlängerung der ersten Aufnahme oder der zweiten Aufnahme hervorsteht, derart, dass sich der Aufnahmeraum und das Dosimeter zwischen der ersten Aufnahme oder der zweiten Aufnahme und den Augen des Nutzers befinden,
wobei das erste Seitenteil oder das zweite Seitenteil einen Befestigungsbereich (40) für das Dosimeter aufweisen, **dadurch gekennzeichnet,**
**dass** der Dosimeterhalter vom ersten Seitenteil oder vom zweiten Seitenteil an einem Eckbereich hervorsteht, der durch die Überschneidung einer Innenfläche (29, 129) des Vorderteils und einer Innenfläche (31, 131) des ersten Seitenteils oder des zweiten Seitenteils gebildet wird, und dass der Dosimeterhalter durch ein Befestigungselement abnehmbar an dem Befestigungsbereich befestigt ist und dass der Befestigungsbereich eine innere Ausnehmung (41) aufweist, die sich in einer Innenseite (31, 131) des ersten Seitenteils oder in einer Innenseite des zweiten Seitenteils befindet, wobei die innere Ausnehmung an das erste seitliche Ende (22, 122) oder an das zweite seitliche Ende (24, 124) des Vorderteils angrenzt, wobei der Dosimeterhalter eine Befestigungs-Fußfläche (50) aufweist, die durch das Befestigungselement in der inneren Ausnehmung befestigt ist.

2. Gestell nach Anspruch 1, wobei der Aufnahmeraum eine obere Öffnung (95, 195) zum Einsetzen des Dosimeters und eine untere Entnahmeöffnung (96, 196) zum leichteren Entnehmen des Dosimeters aufweist, wobei die untere Entnahmeöffnung Dimensionen aufweist, die kleiner sind als die Dimensionen des Dosimeters, um zu verhindern, dass das Dosimeter durch die Entnahmeöffnung hindurchgelangt, und um das Dosimeter im Aufnahmeraum zu halten.

3. Gestell nach einem der Ansprüche 1 bis 2, wobei der Aufnahmeraum zwei Seitenwände (92, 93, 192, 193) zum Halten des Dosimeters durch Einklemmen des Dosimeters in dem Aufnahmeraum aufweist.

4. Gestell nach einem der Ansprüche 1 bis 3, wobei der Aufnahmeraum zwei undurchsichtige Seitenwände (192, 193) aufweist.

5. Gestell nach einem der Ansprüche 1 bis 4, wobei der Aufnahmeraum einem Randbereich der ersten oder der zweiten Aufnahme gegenüberliegt, um die Sicht des das Gestell tragenden Nutzers nicht zu behindern.

6. Gestell nach einem der Ansprüche 1 bis 5, wobei der Dosimeterhalter ein erster Dosimeterhalter ist und wobei das Gestell einen zweiten Dosimeterhalter umfasst, der eine zweite Aufnahmekammer zur Aufnahme eines zweiten Dosimeters aufweist, wobei der zweite Dosimeterhalter hinter dem Vorderteil in der Verlängerung der ersten Aufnahme oder der zweiten Aufnahme derart hervorsteht, dass sich die Aufnahmekammer und das zweite Dosimeter zwischen derjenigen der ersten Aufnahme und der zweiten Aufnahme, die den ersten Dosimeterhalter nicht trägt, und den Augen des Nutzers befinden.

7. Gestell nach einem der Ansprüche 1 bis 6, wobei ein Stegteil (8) zum Halten des ersten und des zweiten Mineralglases an einer Außenfläche des Stegbereichs befestigt ist, wobei das Stegteil einen Haltebereich (81) hat, der in Richtung auf die erste und zweite Aufnahme übergeht, um zu verhindern, dass das erste und das zweite Mineralglas sich lösen.

8. Gestell nach einem der Ansprüche 1 bis 7, wobei die erste Aufnahme eine erste Rille (11) und die zweite Aufnahme einer zweite Rille (12, 112) aufweist, wobei die erste Aufnahme ein erstes Mineralglas umfasst, das in der ersten Rille aufgenommen ist, und wobei die zweite Aufnahme ein zweites Mineralglas umfasst, das in der zweiten Rille aufgenommen ist, wobei das erste und das zweite Mineralglas ein zweites zum Absorbieren von ionisierender Strahlung geeignetes Material umfasst.

9. Gestell nach Anspruch 8, wobei die erste Aufnahme eine dritte Rille (13) umfasst, die von der ersten Rille beabstandet ist, und die zweite Aufnahme eine vierte Rille (14, 114) umfasst, die von der zweiten Rille beabstandet ist, wobei die erste Aufnahme ein drittes Glas umfasst, das in der dritten Rille aufgenommen ist, und die zweite Aufnahme ein viertes Glas umfasst, das in der vierten Rille aufgenommen ist, wobei das dritte und das vierte Glas derart positioniert sind, dass sie sich zwischen dem ersten und dem zweiten Glas und den Augen eines Nutzers befinden.

10. Gestell nach Anspruch 9, wobei der Zwischenraum zwischen dem ersten Mineralglas und dem dritten Mineralglas einen ersten Innenraum bildet und der Zwischenraum zwischen dem zweiten Mineralglas und dem vierten Mineralglas einen zweiten Innenraum bildet, wobei der erste und der zweite Innenraum hermetisch sind.

11. Gestell nach einem der Ansprüche 10 bis 11, wobei das dritte Glas und/oder das vierte Glas eine Sehkorrektur aufweisen.

12. Gestell nach einem der Ansprüche 1 bis 11, wobei das erste Seitenteil ein erster Bügel des Gestells (21, 121) und das zweite Seitenteil ein zweiter Bügel des Gestells (23, 123) ist, wobei der erste und der zweite Bügel über ein jeweiliges erstes Gelenk und zweites Gelenk an dem ersten Vorderteil angebracht sind,
wobei das erste Gelenk und das zweite Gelenk ein Kugelgelenk umfassen.

## Claims

1. Frame for glasses for protecting against ionising radiation, the frame (1, 101) comprising a front part (2, 102), a first side part (3, 103) and a second side part (4, 104) extending respectively from a first and a second side end (25, 26, 125, 126) of the front part, wherein the frame comprises a first filler capable of absorbing ionising radiation, wherein the front part comprises a first housing (5, 105) which is intended to carry a first mineral glass and a second housing (6, 106) which is intended to carry a second mineral glass, the first and the second housings being spaced apart from one another by a bridge zone (7, 107) of the front part, wherein the frame comprises a dosimeter door (9, 109), the dosimeter door comprising a receiving chamber (91, 191) intended to receive a dosimeter, the dosimeter door projecting behind the front part in the extension of the first housing or of the second housing, such that the receiving chamber and the dosimeter are located between the first housing or the second housing and the eyes of the user, wherein the first side part or the second side part comprises a fixing zone (40) for the dosimeter door, **characterised in that** the dosimeter door projects from the first side part or from the second side part from an angle zone formed by the intersection of an internal surface (29, 129) of the front part and of an internal surface (31, 131) of the first side part or of the second side part, and the dosimeter door is removably fixed to the fixing zone by a fixing element, and the fixing zone comprises an internal recess (41) located in an internal face (31, 131) of the first side part or an internal face of the second side part, the internal recess being adjacent to the first side end (22, 122) or to the second side end (24, 124) of the front part, the dosimeter door comprising a fixing base (50) which is fixed in the internal recess by the fixing element.

2. Frame according to the claim 1, wherein the receiving chamber comprises an upper opening (95, 195) intended to insert the dosimeter and a lower extraction orifice (96, 196) intended to facilitate the extraction of the dosimeter, the lower extraction orifice having dimensions less than the dimensions of the dosimeter, in order to prevent the dosimeter passing through the extraction orifice and in order to retain the dosimeter in the receiving chamber.

3. Frame according to any one of claims 1 to 2, wherein the receiving chamber comprises two side walls (92, 93, 192, 193) intended to hold the dosimeter in the receiving chamber by clamping the dosimeter.

4. Frame according to any one of claims 1 to 3, wherein the receiving chamber comprises two opaque side walls (192, 193).

5. Frame according to any one of claims 1 to 4, wherein the receiving chamber is located opposite a peripheral zone of the first or second housing, in order to not impede the vision of the user wearing the frame.

6. Frame according to any one of claims 1 to 5, wherein the dosimeter door is a first dosimeter door, and wherein the frame comprises a second dosimeter door, the second dosimeter door comprising a second receiving chamber intended to receive a second dosimeter, the second dosimeter door projecting behind the front part in the extension of the first housing or of the second housing, such that the receiving chamber and the second dosimeter are located between that from among the first housing and the second housing, which does not carry the first dosimeter door and the eyes of the user.

7. Frame according to any one of claims 1 to 6, wherein a bridge part (8) intended to retain the first and second mineral glasses is fixed on an external face of the bridge zone, wherein the bridge part comprises a retaining portion (81), which overflows towards the first and second housings to prevent the first and second mineral glasses dislodging.

8. Frame according to any one of claims 1 to 7, wherein the first housing comprises a first recess (11) and the second housing comprises a second recess (12, 112), wherein the first housing comprises a first mineral glass which is housed in the first recess, and wherein the second housing comprises a second mineral glass which is housed in the second recess, the first and the second mineral glasses comprising a second filler capable of absorbing ionising radiation.

9. Frame according to claim 8, wherein the first housing comprises a third recess (13) spaced apart from the first recess and the second housing comprises a fourth recess (14, 114) spaced apart from the second recess, wherein the first housing comprises a third glass which is housed in the third recess and the second housing comprises a fourth glass which is housed in the fourth recess, the third and fourth glasses being positioned, so as to be located between the first and second glasses and the eyes of a user.

10. Frame according to claim 9, wherein the spacing located between the first mineral glass and the third glass forms a first internal space, and wherein the spacing located between the second mineral glass and the fourth glass forms a second internal space, wherein the first and second internal spaces are hermetic.

11. Frame according to any one of claims 10 to 11, wherein the third glass and/or the fourth glass has a visual correction.

12. Frame according to any one of claims 1 to 11, wherein the first side part is a first frame arm (21, 121) and the second side part is a second frame arm (23, 123), said first and second arms being attached to the first front part via respectively a first hinge and a second hinge, the first hinge and the second hinge comprising a ball.
